# DEMANDE DE BREVET EUROPEEN

(11) **EP 2 196 216 A1**
(43) Date de publication de la demande: **16.06.2010**
(21) Numéro de dépôt: 10150338.1
(22) Date de dépôt: 24.10.2000
(51) Int. Cl.: A61K 39/39, A61P 11/00, A61P 31/00, A61P 35/00

(54) **Nouvelle composition vaccinale et utilisation d'agents tensioactifs comme adjuvants d'immunité**

(30) Priorité: 29.10.1999 FR 9913618
(62) Demande divisionnaire de: 00402936.9
(71) Demandeur: Société d'Exploitation de Produits pour les Industries Chimiques SEPPIC, 75007 Paris (FR)
(72) Inventeur: Ganne, Vincent, 94210, La Varenne Saint Hilaire (FR); Aucouturier, Jérôme, 92390, Bourg La Reine (FR); Trouve, Gérard, 81100, Castres (FR)
(74) Mandataire: Conan, Philippe Claude

(57) **Abrégé**

L'invention a pour objet une nouvelle composition aqueuse comprenant au moins un antigène ou au moins un générateur in vivo d'un composé comprenant une séquence d'acides aminés, et à titre d'adjuvant d'immunité un agent tensioactif ou un mélange d'agents tensioactifs, ayant un nombre HLB global compris entre 5 et 15, son utilisation comme médicament et l'utilisation dudit tensioactif comme adjuvant d'immunité.

## Description

La présente invention concerne de nouveaux adjuvants pour compositions vaccinales ainsi que des compositions comprenant au moins un antigène, notamment un antigène d'origine virale, bactérienne ou parasitaire et au moins un adjuvant.

Le développement de vaccins inactivés ou contenant des antigènes purifiés est de plus en plus important, car il permet d'éviter les effets secondaires indésirables. Cependant, l'amélioration de la qualité des antigènes se fait au détriment de leur caractère immunogène. C'est pour cette raison qu'ils sont associés à des adjuvants d'immunité.

Les adjuvants d'immunité sont des produits qui augmentent les réactions du système immunitaire, lorsqu'ils sont administrés en présence d'antigènes d'origine virale, bactérienne ou synthétique. Ils provoquent l'apparition massive de macrophages au site d'injection, puis dans les nodules lymphatiques, accroissent la production d'immunoglobulines spécifiques, les anticorps, et stimulent de nombreuses cellules impliquées dans les mécanismes de la défense immunitaire.

Ces adjuvants sont de natures diverses. Ils peuvent par exemple consister en des liposomes ou des émulsions.

Les adjuvants de Freund sont très efficaces ; ils résultent de l'association d'une huile minérale et d'un ester de mannitol contenant ou non une mycobactérie tuée. Les vaccins réalisés par mélange en part égale d'un adjuvant de Freund avec un milieu antigénique aqueux, restent encore les références dans le monde entier pour les études en laboratoire. Ils se présentent sous forme d'émulsions eau dans huile (E/H), c'est à dire d'émulsions dans lesquelles la phase continue est l'huile. Ces émulsions sont très visqueuses ; elles sont donc difficilement injectables ; elles sont aussi peu stables, puisque des déphasages sont observés quelques jours seulement, après leur préparation.

Comme adjuvants usuels, il y a aussi des sels de métaux, tels que l'hydroxyde d'aluminium, le nitrate de cérium, le sulfate de zinc, l'hydroxyde de fer colloïdal ou le chlorure de calcium. Parmi ceux-ci, l'hydroxyde d'aluminium est le plus couramment utilisé. Ces adjuvants sont décrits dans l'article de Rajesh K. Gupta et al "Adjuvants, balance between toxicity and adjuvanticity", Vaccine, vol. 11, Issue 3, 1993, pages 993-306. Ils présentent cependant une faible efficacité immunostimulante et induisent parfois, lorsque ces compositions thérapeutiques sont injectées, la formation de lésions et autres réactions locales, telles que les granulomes, au point d'injection.

Plus récemment, il a été découvert que les sels hydrosolubles de métaux divalents ou trivalents étaient de bons adjuvants de l'immunité notamment le gluconate de manganèse, le gluconate de calcium, le glycérophosphate de manganèse, l'acétate d'aluminium soluble ou le salicylate d'aluminium. De tels adjuvants sont décrits dans les demandes internationales de brevet publiées sous les numéros WO 96/32964 et WO 98/17311.

Comme autres adjuvants de l'immunité, notamment dans le cas de l'administration mucosale, on peut citer les composés sympathomimétiques décrits dans la demande internationale de brevet publiée sous le numéro WO 98/15288.

A l'occasion de ses recherches sur la mise au point de nouveaux adjuvants, la demanderesse a découvert, que certains agents tensioactifs eux-mêmes, possédaient une efficacité immunostimulante et que l'on pouvait ainsi préparer des compositions vaccinales aqueuses essentiellement exemptes de phase huileuse, comprenant un ou plusieurs de ces agents comme immunostimulant.

C'est pourquoi la présente invention a pour objet, une composition sous forme d'une solution aqueuse comprenant :
- (i) au moins un antigène ou au moins un générateur in vivo d'un composé comprenant une séquence d'acides aminés et,
- (ii) à titre d'adjuvant d'immunité un agent tensioactif ou un mélange d'agents tensioactifs choisi parmi un dérivé alkoxylé d'huile de maïs ou un mélange de dérivés alkoxylés d'huile de maïs, ayant un nombre HLB global compris entre 10 et 14, étant entendu que le nombre HLB est différent de 10.

L'invention a également pour objet l'utilisation d'un agent tensioactif ou un mélange d'agents tensioactifs, choisis parmi les dérivés alkoxylés d'huile de maïs, ayant un nombre HLB global compris entre 12 et 13,5, et plus particulièrement parmi les dérivés éthoxylés d'huile de maïs ayant un nombre d'OE compris entre 20 et 40, pour la fabrication d'un adjuvant d'immunité dans une composition sous forme de solution aqueuse comprenant au moins un antigène ou au moins un générateur in vivo d'un composé comprenant une séquence d'acides aminés.

Par antigène ou au moins un générateur in vivo d'un composé comprenant une séquence d'acides aminés, on désigne soit des micro-organismes tués, tels que les virus, les bactéries ou les parasites, soit des fractions purifiées de ces micro-organismes, soit des micro-organismes vivants dont le pouvoir pathogène a été atténué. A titre de virus pouvant constituer un antigène selon la présente invention, on peut citer le virus de la rage, les herpès virus, tels que le virus de la maladie d'Aujeszky, les orthomixovirus tels que Influenzae, les picornavirus tels que le virus de la fièvre aphteuse ou les rétrovirus tels que les VIH. A titre de micro-organisme du type bactérien pouvant constituer un antigène selon la présente invention, on peut citer E. Coli, et ceux des genres Pasteurella, Furonculosis, Vibriosis, Staphylococcus et Streptococcus. A titre de parasite, on peut citer ceux des genres Trypanosoma, Plasmodium et Leishmania. On peut aussi citer les virus recombinants notamment les virus non enveloppés tels que les adénovirus, le virus de la vaccine, le virus Canarypox, les herpès virus ou les baculovirus. On désigne aussi un vecteur recombinant viral non enveloppé vivant, dont le génome contient, insérée de préférence dans une partie non essentielle pour la réplication du virus enveloppé correspondant, une séquence codant pour une sous-unité antigénique induisant une synthèse d'anticorps et/ou un effet protecteur contre le susdit virus enveloppé ou micro-organisme pathogène ; ces sous-unités antigéniques peuvent être par exemple, une protéine, une glycoprotéine, un peptide ou une fraction peptidique et/ou protectrice contre une infection par un micro-organisme vivant tel un virus enveloppé, une bactérie ou un parasite. Le gène exogène inséré dans le micro-organisme peut être, par exemple, issu d'un virus Aujeszky ou HIV.

On peut citer notamment un plasmide recombinant constitué d'une séquence de nucléotides, dans laquelle est insérée une séquence nucléotidique exogène, provenant d'un micro-organisme ou d'un virus pathogène. Cette dernière séquence nucléotidique a pour but de permettre l'expression d'un composé comprenant une séquence d'acides aminés, ce composé ayant lui-même pour but de déclencher une réaction immune dans un organisme hôte.

Par générateur "in vivo" d'un composé comprenant une séquence d'acides aminés, on désigne tout un produit biologique capable d'exprimer ledit composé dans l'organisme hôte dans lequel on a introduit ledit générateur in vivo. Le composé comprenant la séquence d'acides aminés, peut être une protéine, un peptide ou une glycoprotéine. Ces générateurs in vivo sont généralement obtenus par des procèdes issus du génie génétique. Plus particulièrement, ils peuvent consister en des micro-organismes vivants, généralement un virus, jouant le rôle de vecteur recombinant, dans lequel est insérée une séquence nucléotidique, notamment un gène exogène. Ces composés sont connus en tant que tels et utilisés notamment comme vaccin sous unitaire recombinant. A cet égard, on peut se référer à l'Article de M. ELOIT et al., Journal of virology (1990) 71, 2925-2431 et aux demandes internationales de brevet publiées sous les numéros WO-A-91/00107 et WO-A-94/16681. Les générateurs in vivo selon l'invention peuvent aussi consister en un plasmide recombinant comprenant une séquence nucléotidique exogène, capable d'exprimer dans un organisme hôte un composé comprenant une séquence d'acides aminés. De tels plasmides recombinants et leur mode d'administration dans un organisme hôte ont été décrits en 1990, par LIN et al., Circulation 82:2217,2221 ; COX et al., J. of VIROL, Sept. 1993, 67, 9, 5664-5667 et dans la demande internationale publiée sous le numéro WO 95/25542. Selon la nature de la séquence nucléotidique comprise dans le générateur in vivo, le composé comprenant la séquence d'acides aminés qui est exprimé au sein de l'organisme hôte, peut :
(i) être un antigène, et permettre le déclenchement d'une réaction immune,
(ii) avoir une action curative vis-à-vis d'une maladie, essentiellement une maladie d'ordre fonctionnel, qui s'est déclenchée chez l'organisme hôte. Dans ce cas, le générateur in vivo permet un traitement de l'hôte, du type thérapie génique.

A titre d'exemple, une telle action curative peut consister en une synthèse par le générateur in vivo de cytokines, comme les interleukines, notamment l'interleukine 2. Celles-ci permettent le déclenchement ou le renforcement d'une réaction immune visant à l'élimination sélective des cellules cancéreuses.

Une composition selon l'invention comprend une concentration en antigène qui dépend de la nature de cet antigène et de la nature du sujet traité. Il est toutefois particulièrement remarquable qu'un adjuvant selon l'invention, permette de diminuer notablement la dose habituelle d'antigène requise. La concentration adéquate d'antigène peut être déterminée de manière classique par l'homme du métier. Généralement, cette dose est de l'ordre de 0,1µg/cm³ à 1g/cm³ plus généralement comprise entre 1µg/cm³ et 100mg/cm³.

La concentration dudit générateur in vivo dans la composition selon l'invention dépend, là encore, notamment de la nature dudit générateur et de l'hôte dans lequel il est administré. Cette concentration peut être aisément déterminée par l'homme du métier, sur la base d'expérience de routine. A titre indicatif, on peut toutefois préciser que lorsque le générateur in vivo est un microorganisme recombinant, sa concentration dans la composition selon l'invention peut être comprise entre 10² et 10¹⁵ micro-organismes/cm³, de préférence entre 10⁵ et 10¹² micro-organismes/cm³. Lorsque le générateur in vivo est un plasmide recombinant, sa concentration dans la composition selon l'invention peut être comprise entre 0,01 et 100 g/dm³.

Au sens de la présente invention, le nombre HLB est calculé par la formule HLB = 20 (1-Iₛ/Iₐ), dans laquelle Iₛ représente l'indice de saponification et Iₐ, l'indice d'acide dudit tensioactif ou dudit mélange d'agents tensioactifs. Ces deux indices, de saponification et d'acide, sont déterminés par des méthodes décrites dans la Pharmacopée européenne.

Une composition objet de la présente invention, contient entre 0,2mg/cm³ et 500mg/cm³ d'adjuvant, plus particulièrement entre 2mg/cm³ et 500mg/cm³ d'adjuvant et de préférence entre 50mg/cm³ et 200mg/cm³ d'adjuvant.

Un agent tensioactif selon l'invention est de préférence pharmaceutiquement acceptable au niveau des muqueuses ; il doit notamment être dépourvu de métaux lourds et présenter des indices d'acides ou de peroxydes très faibles. Il est également souhaitable qu'il satisfasse aux normes des tests d'innocuité tels que, ceux décrits par S.S. Berllin, Annales of Allergy, 1962, 20, 473 ou les tests de toxicité anormale décrits dans la Pharmacopée européenne.

La composition selon l'invention, peut comporter un agent stimulant immunitaire conventionnel tel l'Avridine®, la N,N-dioctadecyl-N',N'-bis(2-hydroxyethyl) propanediamine, les dérivés du MDP (muramyl dipeptide), notamment le thréonyl-MDP, les dérivés de l'acide mycolique ou les dérivés du Lipide A.

La composition selon l'invention, peut comprendre un ou plusieurs sels organiques de cations métalliques hydrosolubles, tel que par exemple le gluconate de calcium, le gluconate de manganèse, le salicylate d'aluminium ou l'acétate d'aluminium soluble. Lorsque la composition adjuvante selon l'invention comprend, un sel pharmaceutiquement acceptable, celui-ci est à une concentration de 0,02 à 3000 mg/cm³, de préférence 0,1 à 1000 mg/cm³, plus préférentiellement de 0,1 à 150 mg/cm³.

La composition selon l'invention, peut comporter un composé sympathomimétique. Par composés sympathomimétiques, on désigne notamment les amphétamines, les catécholamines, les phénylisopropylamines ou la tyramine. Comme exemples de tels composés, on peut citer notamment l'isoprotérénol, la L-Epinephrine, le lévartérénol, l'éphédrine, la phényléphédrine ou le salbutamol. Lorsque la composition adjuvante selon l'invention comprend un composé sympathomimétique, celui-ci à une concentration de 10⁻¹⁰ molaire à 10⁻² molaire, de préférence de 10⁻⁷ Molaire à 10⁻⁵ Molaire.

L'utilisation des agents tensioactifs tels que définis précédemment comme adjuvant dans les compositions vaccinales et plus particulièrement dans les compositions vaccinales sans phase huileuse constitue un autre aspect de la présente invention.

La composition selon l'invention peut être utilisée comme médicament préventif ou curatif. Selon la nature de l'antigène ou du générateur in vivo, une composition selon l'invention peut être administrée à des poissons, des crustacés tels que les crevettes, des volailles, notamment, des oies, des dindes, des pigeons et des poulets, aux canidés tels le chien, aux félidés tels le chat, aux porcs, aux primates, aux bovidés, aux ovidés et aux chevaux. La composition selon l'invention peut être également administrée à l'homme. L'administration de la composition peut se faire de manière classique par voie parentérale, notamment par injection sous-cutanée, intramusculaire ou intrapéritonéale ou par voie mucosale notamment par voie orale, voie rectale, voie nasale, voie vaginale.

Selon un autre aspect de l'invention, celle-ci consiste en l'utilisation d'un adjuvant tel que défini ci-dessus pour la préparation d'un vaccin destiné à la prévention ou au traitement d'une maladie infectieuse, notamment une maladie infectieuse engendrée par un virus ou un micro-organisme tels ceux mentionnés plus haut. Selon un autre dernier aspect de la présente invention, celle-ci consiste en l'utilisation de cet adjuvant pour la préparation d'une composition destinée à soigner une maladie d'ordre fonctionnel, telle le cancer ou la mucoviscidose.

### Exemple 1

On a injecté par voie sous-cutanée, à différents lots de 5 souris femelles de souche OF1 ayant un poids moyen de 18 à 20 grammes, 100 microlitres de différentes compositions contenant un agent tensioactif, du tampon phosphate (PBS) et 10 mg/cm³ d'ovalbumine, à t = 0 avec un rappel à t = 28 jours.

On effectue les prélèvements sanguins à 14, 28, 42, 56, 90 et 180 jours.

On procède à des dosages ELISA sur les prélèvements sanguins, des IgG1 pour déterminer la réponse immunitaire humorale et les IgG2a, pour déterminer la réponse immunitaire cellulaire. Les réactions locales sont évaluées à 7 jours et à 35 jours.

Les compositions sont les suivantes :

| Tensioactif utilisé (TA) (Composition) % pondéraux | HLB du TA | TA en µl | Tampon (PBS) en µl | Antigène (10mg/cm³) en µl |
|---|---|---|---|---|
| Huile de maïs éthoxylée (30E) (Référence 1) | 4,1 | 100 | 1900 | 20 |
| Huile de maïs éthoxylée (10 OE) + 2 % glycérol sur charge initiale (Composition A) | 7,9 | 100 | 1900 | 20 |
| Huile de maïs éthoxylée (20 OE) + 2 % glycérol sur charge initiale (Composition B) | 10,4 | 100 | 1900 | 20 |
| Huile de maïs éthoxylée (30 OE) + 2 % glycérol sur charge initiale (Composition C) | 12,3 | 100 | 1900 | 20 |
| Huile de maïs éthoxylée (40 OE) + 2 % glycérol sur charge initiale (Composition D) | 13,8 | 100 | 1900 | 20 |
| Huile de maïs éthoxylée (20 OE) + 4 % glycérol sur charge initiale (Composition E) | 14,2 | 100 | 1900 | 20 |
| Huile de maïs éthoxylée (40 OE) + 4 % glycérol sur charge initiale (Composition F) | 11,3 | 100 | 1900 | 20 |
| Oléate de mannitane (50E) (Composition G) | 10,9 | 100 | 1900 | 20 |
| Oléate de mannitane (80E) (Composition H) | 12,4 | 100 | 1900 | 20 |
| Oléate de mannitane (100E) (Composition I) | 13,1 | 100 | 1900 | 20 |
| Oléate de mannitane (150E) (Composition J) | 14,6 | 100 | 1900 | 20 |
| Oléate de mannitane (200E) (Composition K) | 15,6 | 100 | 1900 | 20 |
| Oléate de mannitane (400E) (Composition L) | 17,3 | 100 | 1900 | 20 |
| Oléate de mannitane (80E) (Composition H) | 12,4 | 100 | 1900 | 20 |
| Oléate de mannitane (100E) (Composition I) | 13,1 | 100 | 1900 | 20 |
| Oléate de mannitane (150E) (Composition J) | 14,6 | 100 | 1900 | 20 |
| Oléate de mannitane (200E) (Composition K) | 15,6 | 100 | 1900 | 20 |
| Oléate de mannitane (400E) (Composition L) | 17,3 | 100 | 1900 | 20 |
| Oléate de mannitane (80E) (Composition H) | 12,4 | 100 | 1900 | 20 |

Les résultats des tests ELISA sont les suivants :

| | **Dosage des IG1 (échelle de temps en jours)** | | | | | |
|---|---|---|---|---|---|---|
| **Composition** | **J14** | **J28** | **J42** | **J56** | **J90** | **J180** |
| Référence (1) | 1500 | 1000 | 32000 | 48000 | 32000 | 6000 |
| Composition (A) | 1000 | 1000 | 8000 | 12000 | 3000 | 1500 |
| Composition (B) | 1000 | 1000 | 64000 | 64000 | 16000 | 8000 |
| Composition (C) | 2000 | 1000 | 96000 | 128000 | 128000 | 12000 |
| Composition (D) | 1500 | 1000 | 6000 | 32000 | 64000 | 6000 |
| Composition (E) | 1000 | 1000 | 32000 | 64000 | 96000 | 24000 |
| Composition (F) | 3000 | 8000 | 64000 | 128000 | 128000 | 32000 |
| Composition (G) | 2000 | 2000 | 8000 | 64000 | 48000 | 8000 |
| Composition (H) | 4000 | 8000 | 128000 | 128000 | 48000 | 16000 |
| Composition (I) | 4000 | 1000 | 128000 | 96000 | 48000 | 12000 |
| Composition (J) | 1000 | 2000 | 64000 | 24000 | 8000 | 2000 |
| Composition (K) | 1000 | 1000 | 24000 | 12000 | 2000 | 2000 |
| Composition (L) | 1000 | 1000 | 18000 | 6000 | 2000 | 2000 |
| Référence (2) | 1000 | 1000 | 32000 | 16000 | 3000 | 2000 |
| Composition (M) | 4000 | 4000 | 128000 | 128000 | 256000 | 48000 |
| Composition (N) | 1500 | 1000 | 128000 | 64000 | 32000 | 12000 |
| Composition (O) | 1000 | 1000 | 32000 | 20000 | 16000 | 2000 |
| Référence (3) | 32000 | 32000 | 256000 | 128000 | 32000 | 8000 |
| Témoin | 1000 | 1000 | 4000 | 2000 | 3000 | 1000 |

| | | | | | | |
|---|---|---|---|---|---|---|
| | **Dosage des IG2 (échelle de temps en jours)** | | | | | |
| **Composition** | **J14** | **J28** | **J42** | **J56** | **J90** | **J180** |
| Référence (1) | 1000 | 1000 | 1000 | 1500 | 3000 | 1000 |
| Composition (A) | 1000 | 1000 | 1000 | 1000 | 2000 | 1000 |
| Composition (B) | 1000 | 1000 | 2000 | 2000 | 2000 | 1000 |
| Composition (C) | 1000 | 1000 | 4000 | 1500 | 3000 | 1000 |
| Composition (D) | 1000 | 1000 | 1000 | 2000 | 4000 | 1000 |
| Composition (E) | 1000 | 1000 | 1000 | 2000 | 3000 | 1000 |
| Composition (F) | 1000 | 1000 | 1000 | 6000 | 4000 | 1000 |
| Composition (G) | 1000 | 1000 | 1000 | 8000 | 3000 | 1000 |
| Composition (H) | 1000 | 1000 | 1000 | 3000 | 3000 | 1000 |
| Composition (I) | 1000 | 1000 | 4000 | 3000 | 2000 | 1000 |
| Composition (J) | 1000 | 1000 | 3000 | 1500 | 2000 | 1000 |
| Composition (K) | 1000 | 1000 | 1000 | 1500 | 2000 | 1000 |
| Composition (L) | 1000 | 1000 | 1000 | 1500 | 2000 | 1000 |
| Référence (2) | 1000 | 1000 | 1000 | 1500 | 3000 | 1000 |
| Composition (M) | 1000 | 1000 | 4000 | 1500 | 6000 | 1000 |
| Composition (N) | 1000 | 1000 | 4000 | 1500 | 2000 | 1000 |
| Composition (O) | 1000 | 1000 | 4000 | 1500 | 6000 | 1000 |
| Référence (3) | 1000 | 1000 | 4000 | 16000 | 2000 | 1000 |
| Témoin | 1000 | 1000 | 1000 | 1000 | 1000 | 1000 |

### Exemple 2

On opère de la même manière qu'à l'exemple 1 avec comme tensioactifs, les huiles de ricin éthoxylées suivantes :

| Tensioactif utilisé (TA) (Composition) % pondéraux | HLB du TA | TA en µl | Tampon (PBS) en µl | Antigène (10mg/cm³) en µl |
|---|---|---|---|---|
| Huile de ricin éthoxylée : 100 % (70E) (Composition P) | 6 | 100 | 1900 | 20 |
| Huile de ricin éthoxylée : 89,13 % (7 OE) + 10,87 % (60 OE) (Composition Q) | 7 | 100 | 1900 | 20 |
| Huile de ricin éthoxylée : 78,26 % (7 OE) + 21,74 % (60 OE) (Composition R) | 8 | 100 | 1900 | 20 |
| Huile de ricin éthoxylée : 67,39 % (7 OE) + 32,61 % (60 OE) (Composition S) | 9 | 100 | 1900 | 20 |
| Huile de ricin éthoxylée : 56,52 % (7 OE) + 43,487 % (60 OE) (Composition T) | 10 | 100 | 1900 | 20 |
| Huile de ricin éthoxylée : 50 % (7 OE) + 50 % (60 OE) (Composition U) | 10,6 | 100 | 1900 | 20 |
| Huile de ricin éthoxylée : 45,65 % (7 OE) + 54,35 % (60 OE) (Composition V) | 11 | 100 | 1900 | 20 |
| Huile de ricin éthoxylée : 34,78 % (7 OE) + 65,22 % (60 OE) (Composition W) | 12 | 100 | 1900 | 20 |
| Huile de ricin éthoxylée : 23,91 % (7 OE) + 76,09 % (60 OE) (Composition X) | 13 | 100 | 1900 | 20 |
| Huile de ricin éthoxylée : 13,04 % (7 OE) + 86,96 % (60 OE) (Composition Y) | 14 | 100 | 1900 | 20 |
| Huile de ricin éthoxylée : 100 % (60E) (composition Z) | 15,2 | 100 | 1900 | 20 |
| Témoin (T1) | | 1000 | 1000 | 20 |
| Témoin (T2) | | 0 | 2000 | 20 |

Les résultats des tests ELISA sont les suivants :

| | **Dosage des IG1 (échelle de temps en jours)** | | | | | |
|---|---|---|---|---|---|---|
| **Composition** | **J14** | **J28** | **J42** | **J56** | **J90** | **J180** |
| Composition (P) | 1600 | 600 | 8000 | 16000 | 12000 | nd |
| Composition (Q) | 3200 | 600 | 16000 | 64000 | 48000 | nd |
| Composition (R) | 2400 | 400 | 48000 | 64000 | 64000 | nd |
| Composition (S) | nd | nd | nd | nd | nd | nd |
| Composition (T) | 600 | 100 | 16000 | 32000 | 32000 | nd |
| Composition (U) | 100 | 100 | 8000 | 32000 | 32000 | nd |
| Composition (V) | 100 | 100 | 8000 | 12000 | 6000 | nd |
| Composition (W) | nd | nd | nd | nd | nd | nd |
| Composition (X) | 200 | 100 | 3000 | 4000 | 3000 | nd |
| Composition (Y) | 400 | 100 | 4000 | 12000 | 8000 | nd |
| Composition (Z) | 100 | 100 | 8000 | 6000 | 6000 | nd |
| témoin T1 | 19200 | 12800 | 256000 | 128000 | 128000 | nd |
| Témoin T2 | 100 | 100 | 4000 | 2000 | 1500 | nd |

| | **Dosage des IG2 (échelle de temps en jours)** | | | | | |
|---|---|---|---|---|---|---|
| **Composition** | **J14** | **J28** | **J42** | **J56** | **J90** | **J180** |
| Composition (P) | 100 | 100 | 8000 | 3000 | 3000 | nd |
| Composition (Q) | 100 | 100 | 12000 | 4000 | 8000 | nd |
| Composition (R) | 100 | 100 | 32000 | 8000 | 8000 | nd |
| Composition (S) | nd | nd | nd | nd | nd | nd |
| Composition (T) | 100 | 100 | 16000 | 3000 | 8000 | nd |
| Composition (U) | 100 | 100 | 6000 | 4000 | 4000 | nd |
| Composition (V) | 100 | 100 | 4000 | 15000 | 2000 | nd |
| Composition (W) | nd | nd | nd | nd | nd | nd |
| Composition (X) | 100 | 100 | 1000 | 1000 | 1000 | nd |
| Composition (Y) | 100 | 100 | 1000 | 1000 | 1000 | nd |
| Composition (Z) | 100 | 100 | 1000 | 1000 | 3000 | nd |
| témoin T1 | 100 | 100 | 8000 | 3000 | 8000 | nd |
| Témoin T2 | 100 | 100 | 1000 | 1000 | 1000 | nd |

## Revendications

1. Utilisation d'un agent tensioactif ou un mélange d'agents tensioactifs, choisis parmi les dérivés alkoxylés d'huile de maïs, ayant un nombre HLB global compris entre 12 et 13,5, et plus particulièrement parmi les dérivés éthoxylés d'huile de maïs ayant un nombre d'OE compris entre 20 et 40, pour la fabrication d'un adjuvant d'immunité dans une composition sous forme de solution aqueuse comprenant au moins un antigène ou au moins un générateur in vivo d'un composé comprenant une séquence d'acides aminés.

2. Composition sous forme d'une solution aqueuse comprenant :
• (i) au moins un antigène ou au moins un générateur in vivo d'un composé comprenant une séquence d'acides aminés et,
• (ii) à titre d'adjuvant d'immunité un agent tensioactif ou un mélange d'agents tensioactifs choisi parmi un dérivé alkoxylé d'huile de maïs ou un mélange de dérivés alkoxylés d'huile de maïs, ayant un nombre HLB global compris entre 10 et 14, étant entendu que le nombre HLB est différent de 10.

3. Composition telle que définie à la revendication 2, dans laquelle l'adjuvant d'immunité est un agent tensioactif ou un mélange d'agents tensioactifs choisi parmi les dérivés éthoxylés d'huile de maïs ayant un nombre d'OE compris entre 20 et 40.

4. Composition telle que définie à l'une des revendications 2 ou 3, comprenant en outre un ou plusieurs sels organiques de cations métalliques hydrosolubles, tels que le gluconate de calcium, le gluconate de manganèse, le salicylate d'aluminium ou l'acétate d'aluminium soluble.

5. Composition selon l'une des revendications 2 à 4, pour la mise en oeuvre d'une méthode de traitement du corps humain ou animal.
